# EUROPEAN PATENT APPLICATION

(11) **EP 2 184 070 A1**
(43) Date of publication of application: **12.05.2010**
(21) Application number: 08168675.0
(22) Date of filing: 07.11.2008
(51) Int. Cl.: A61K 38/17, A61K 47/48, C07K 14/705, C12N 15/62, C12N 15/79, C12N 5/10, A61P 37/06, A61P 29/00

(54) **HLA-G proteins and pharmaceutical uses thereof**

(71) Applicant: Hla-G Technologies, 69005 Lyon (FR); Commissariat à l'Energie Atomique, 75015 Paris (FR)
(72) Inventor: Favier, Benoit, 75019, PARIS (FR); Carosella, Edgardo D., 75016, PARIS (FR); Lemaoult, Joël, 77000, MELUN (FR)
(74) Representative: Becker, Philippe

(57) **Abstract**

The present invention relates to novel proteins and pharmaceutical uses thereof. The invention more specifically relates to novel fusion proteins comprising a domain of an HLA-G antigen fused to an Fc domain of an immunoglobulin. The invention also relates to methods of producing such polypeptides, pharmaceutical compositions comprising the same, as well as their uses for treating various diseases including organ/tissue rejection.

## Description

The present invention relates to novel proteins and pharmaceutical uses thereof. The invention more specifically relates to novel fusion proteins comprising a domain of an HLA-G antigen fused to an Fc domain of an immunoglobulin. The invention also relates to methods of producing such polypeptides, pharmaceutical compositions comprising the same, as well as their uses for treating various diseases including organ/tissue rejection.

### BACKGROUND

Major histocompatibility complex (MHC) antigens are divided up into three main classes, namely class I antigens, class II antigens (HLA-DP, HLA-DQ and HLA-DR), and class III antigens.

Class I antigens comprise conventional antigens, HLA-A, HLA-B and HLA-C, which exhibit 3 globular domains ([alpha]1, [alpha]2 and [alpha]3), as well as unconventional antigens HLA-E, HLA-F, and HLA-G.

HLA-G is a non-classic HLA Class I molecule expressed by extravillous trophoblasts of normal human placenta and thymic epithelial cells. HLA-G antigens are essentially expressed by the cytotrophoblastic cells of the placenta and function as immunomodulatory agents protecting the foetus from the maternal immune system (absence of rejection by the mother). The sequence of the HLA-G gene has been described (e.g., Geraghty et al. Proc. Natl. Acad. Sci. USA, 1987, 84, 9145-9149 ; Ellis; et al., J. Immunol., 1990, 144, 731-735) and comprises 4396 base pairs. This gene is composed of 8 exons, 7 introns and a 3' untranslated end, corresponding respectively to the following domains: exon 1: signal sequence, exon 2: alpha1 extracellular domain, exon 3: alpha2, extracellular domain, exon 4: alpha3 extracellular domain, exon 5: transmembrane region, exon 6: cytoplasmic domain I, exon 7: cytoplasmic domain II (untranslated), exon 8: cytoplasmic domain III (untranslated) and 3' untranslated region. Seven isoforms of HLA-G have been identified, among which 4 are membrane bound (HLA-G1, HLA-G2, HLA-G3 and HLA-G4) and 3 are soluble (HLA-G5, HLA-G6 and HLA-G7) (see e.g., Carosella et al., Blood 2008, vol. 111, p 4862).

The mature HLA-G1 protein isoform comprises the three external domains (α1-α3), the transmembrane region and the cytoplasmic domain.
The HLA-G2 protein isoform does not comprise the α2 domain, i.e., the α1 and α3 domains are directly linked, followed by the transmembrane domain and the cytoplasmic domain.
The HLA-G3 protein isoform lacks both the α3 and α3 domains, i.e., it comprises the α1 domain directly linked to the transmembrane domain and the cytoplasmic domain. The HLA-G4 protein isoform lacks the α3 domain, i.e., it comprises the α1 domain, the α2 domain, the transmembrane domain and the cytoplasmic domain.

Soluble HLA-G isoforms all lack the transmembrane and cytoplasmic domains. More specifically:
The HLA-G5 protein isoform contains the α1, α2 and α3 domains, as well as an extra C-terminal peptide sequence of 21 amino acid residues encoded by intron 4 (as a result of intron 4 retention after transcript splicing and RNA maturation).
The HLA-G6 protein isoform corresponds to the HLA-G5 without α2, i.e., HLA-G6 contains α1 and α3 domains, as well as an extra C-terminal peptide sequence of 21 amino acid residues encoded by intron 4 (as a result of intron 4 retention after transcript splicing and RNA maturation
The HLA-G7 protein isoform contains only the alpha1 domain, as well as 2 additional C-terminal amino acid residues encoded by intron2 (as a result of intron 2 retention after transcript splicing and RNA maturation).

All of these isoforms have been described e.g., in Kirszenbaum M. et al., Proc. Natl. Acad. Sci. USA, 1994, 91, 4209-4213; European Application EP 0 677 582; Kirszenbaum M. et al., Human Immunol., 1995, 43, 237-241; Moreau P. et al., Human Immunol., 1995, 43, 231-236).

Previous studies have shown that HLA-G proteins are able to inhibit allogeneic responses such as proliferative T lymphocyte cell response, cytotoxic T lymphocytes mediated cytolysis, and NK cells mediated cytolysis (Rouas-Freiss N. et al., Proc. Natl. Acad. Sci., 1997, 94, 5249-5254 ; Semin Cancer Biol 1999, vol 9, p. 3). As a result, HLA-G proteins have been proposed for treating graft rejection in allogeneic or xenogenic organ/tissue transplantation. HLA-G proteins have also been proposed for the treatment of cancers (EP1 054 688), inflammatory disorders (EP1 189 627) and, more generally, immune related diseases. It has also been proposed to fuse HLA-G proteins to specific ligands in order to target HLA-G to particular cells or tissues (WO2007091078). It should be noted, however, that no results or experimental data have been provided to show that such targeting fusions are active.

HLA-G isoforms appear to adopt a dimer conformation as a result of the formation of an intermolecular disulfide bridge between Cysteine residue 42 of the α1 domains of two HLA-G molecules (Apps et al., Eur. J. Immunol. 2007, vol. 37 p. 1924 ; WO2007/011044). It has been proposed that receptor binding sites of HLA-G dimers are more accessible than those of corresponding monomers, so that dimers would have a higher affinity and slower dissociation rate than monomers. However, it is not clear what conformation is the most active for pharmaceutical purpose, especially in relation to soluble forms of HLA-G, nor how appropriate HLA-G dimers or oligomers may be produced.

### SUMMARY OF THE INVENTION

The present invention relates to novel proteins or polypeptides, pharmaceutical compositions comprising the same, and the uses thereof. More specifically, the present invention relates to novel fusion polypeptides comprising an HLA-G-derived sequence and an immunoglobulin Fc fragment-derived sequence. These polypeptides are able to form functionally active dimeric complexes and are able to efficiently inhibit organ rejection in vivo. These polypeptides thus represent drug candidates for treating such disorders, as well as other immune-related diseases.

An object of the present invention thus resides in a fusion polypeptide comprising: a first polypeptide comprising the sequence of a domain of an HLA-G antigen, linked to a second polypeptide comprising the sequence of an Fc domain of an immunoglobulin.

As will be disclosed, the HLA-G domain contained in the fusion polypeptide can comprise all or part of the extracellular portion of an HLA-G antigen, typically all or a functional part of the α1, α2 and/or α3 domains of HLA-G. In a specific embodiment, the Fc domain comprises a sequence that is sufficient to allow the formation of a dimer (homodimer or heterodimer) between two fusion polypeptides of this invention.

In a particular embodiment, the fusion polypeptide of the invention further comprises a third polypeptide domain comprising the sequence of a β2 microglobulin.

Another object of this invention is a premature form of a polypeptide as described above, further comprising a signal peptide sequence causing secretion.

A further object of this invention resides in a nucleic acid molecule encoding a fusion polypeptide as disclosed above.

The invention also relates to a vector comprising a nucleic acid molecule as defined above.

Another object of this invention is a recombinant host cell comprising a nucleic acid molecule or a vector as defined above.

A further object of this invention is a method of producing a polypeptide as defined above, comprising culturing a recombinant host cell of the invention under conditions allowing expression of the nucleic acid molecule, and recovering the polypeptide produced.

The invention further relates to a dimer (e.g., a homodimer or a heterodimer) of a polypeptide of the invention.

The invention also relates to an antibody that specifically binds a fusion polypeptide of this invention or a dimer thereof.

The invention also relates to a pharmaceutical composition comprising a polypeptide as defined above, either as a monomer or as a multimer.

The invention also relates to a pharmaceutical composition comprising a nucleic acid encoding a polypeptide as defined above, or a recombinant cell expressing such a polypeptide.

The invention further relates to such polypeptides or pharmaceutical compositions for treating organ or tissue rejection, inflammatory diseases or auto-immune diseases.

A further objet of this invention also relates to a method of treating organ/tissue rejection, the method comprising administering to a subject in need thereof an effective amount of a polypeptide or composition of this invention. More specifically, the method comprises administering the polypeptide or composition to the subject, prior to, during and/or after tissue/organ transplant.

A further object of this invention is a method of promoting tolerance to graft in a subject, the method comprising administering to a subject in need thereof an effective amount of a polypeptide or composition as defined above.

The invention may be used in any mammalian subject, preferably in human subjects. As will be further disclosed below, the polypeptides of this invention are able to substantially inhibit tissue rejection in vivo following transplantation.

### LEGEND TO THE FIGURES

Figure 1: HLA-G-Fc fusion proteins can form dimers.
Figure 2: HLA-G-Fc fusion proteins induce signalling through ILT2 receptor.
Figure 3: HLA-G6-Fc promotes graft survival in vivo.
Figure 4: HLA-G1-B2M-Fc promotes graft survival in vivo.
Figure 5: HLA-Ga1-Fc promotes graft survival in vivo.

### DETAILED DESCRIPTION OF THE INVENTION

The present invention relates to fusion polypeptides comprising an HLA-G antigen or a portion thereof. The fusion polypeptides of this invention can form biologically active dimers and have been shown to effectively inhibit graft rejection in vivo. More specifically, the inventors have found that, by fusing such an HLA-G antigen to an Fc domain of an immunoglobulin, biologically active proteins can be generated, which have the ability to induce strong immune tolerance and can adopt biologically active conformation in vivo. These results are surprising since HLA-G domains had never been fused to such dimer-forming moities and since the spatial position of HLA-G domains within such dimers differs from that of naturally-occurring HLA-G complexes. The results obtained show that the fusion polypeptides of this invention exhibit high immunoregulatory activity in vivo and therefore represent novel medicaments for treating immune-related disorders, particularly for reducing unwanted or deleterious immune responses in a subject.

A first object of the present invention thus resides in a fusion polypeptide comprising:
- a first polypeptide comprising the sequence of a domain of an HLA-G antigen, linked to
- a second polypeptide comprising the sequence of an Fc domain of an immunoglobulin.

Within the context of the present invention, the terms "polypeptide" and "protein" designate, interchangeably, a molecule comprising a polymer of amino acid residues, which may be linked together through amine linkage, or through modified, peptidomimetic linkages. The amino acid residues in said proteins or polypeptides may be either natural amino acid residues, or non-natural or modified amino acid residues. They may be in L and/or D conformation. Also, the polypeptide or protein may be terminally protected and/or modified, e.g., through chemical or physical alteration of lateral functions, for instance.

Within a fusion polypeptide of this invention, the various polypeptide domains are covalently linked together so that they are, most preferably, produced as a single molecule through recombinant techniques.

Various arrangements are possible within fusion proteins of this invention. In particular, the various domains may be positioned C-ter or N-ter, and linked together either directly or though spacer groups. In a most preferred embodiment, the first polypeptide (i.e., the HLA-G derived sequence) is located N-ter of the second polypeptide (the Fc-derived sequence), which is located C-ter of the fusion polypeptide. As shown in the examples, such conformation allows dimerisation of the polypeptide and efficient biological activity in vivo.

Coupling between the various polypeptide domains can be direct, i.e. with no intervening sequence (although intervening amino acid residues may be present for coupling/cloning purpose or as a result of cloning steps, e.g., corresponding to restriction site(s)), or indirect, i.e., with an intervening sequence (spacer group). In the latter case, the spacer group can have a variable length, typically between 4 and 20 amino acid residues, and should preferably be biologically inert. An example of such spacer group is the (G4S)n motif, with n being an integer from 1 to 4.

In a preferred embodiment of a fusion polypeptide of this invention, the first and second polypeptides are linked directly. More precisely, the C-terminal residue of the HLA-G derived sequence is linked to the N-terminal residue of the Fc-derived sequence.

The HLA-G domain contained in the fusion polypeptide can comprise all or part of the extracellular portion of an HLA-G antigen, typically all or a functional part of the α1, α2 and/or α3 domains of an HLA-G antigen. The amino acid sequence ofHLA-G1 has been described in, e.g, Geraghty et al. or; Ellis; et al., J. Immunol., 1990, 144, 731-735, quoted above. The amino acid sequences of the α1, α2 and α3 domains can be derived directly from said publications. These sequences are also available on line (see for instance Genebank numbers for HLA-G: first cloning of genomic sequence: Geraghty et α1, PNAS 1987: PubMed ID: 3480534, GeneID: 3135 ; First cloning of HLA-G1 cDNA : Ellis et al Journal of Immunology 1990. PubMed ID : 2295808). Furthermore, the sequences of HLA-G5, HLA-G6 and HLA-G7 are also available from US5,856,442, US6,291,659, FR2,810,047, or Paul et al., Hum. Immunol 2000; 61: 1138).

As indicated, the fusion polypeptides of this invention comprise at least a portion of an extracellular domain of an HLA-G antigen. In a preferred embodiment, the HLA-G antigen is a human HLA-G antigen.

In a particular embodiment, the fusion polypeptide of this invention comprises at least the amino acid sequence of the α1 domain of a human HLA-G antigen.

According to other specific embodiments, the first polypeptide of the fusion polypeptides of this invention is selected from:
- the amino acid sequence of the α1, α2 and α3 domains of an HLA-G antigen;
- the amino acid sequence of the α1 and α3 domains of an HLA-G antigen;
- the amino acid sequence of the α1 and α2 domains of an HLA-G antigen ;
- the amino acid sequence of HLA-G5 ;
- the amino acid sequence of HLA-G6 ; or
- the amino acid sequence of HLA-G7.

Specific example of the amino acid sequence of an α1 domain of human HLA-G is provided in SEQ ID NO: 6 (amino acid residues 21 to 110). A specific example of the amino acid sequence of human HLA-G6 is provided in SEQ ID NO: 2 (amino acid residues 25 to 227). A specific example of the amino acid sequence of the α1, α2 and α3 domains of a human HLA-G is provided in SEQ ID NO: 4 (amino acid residues 135-412). It should be understood that natural variants of HLA-G antigens exist, e.g., as a result of polymorphism, which are included in the present application. Also, variants of the above sequences which lack certain (e.g., between 1 and 10, preferably between 1-5, most preferably 1, 2, 3, 4 or 5) amino acid residues, and/or contain certain (e.g., between 1 and 10, preferably between 1-5, most preferably 1, 2, 3, 4 or 5) amino acid substitutions or insertions are also included in the present invention.

The second polypeptide domain of the fusion polypeptides of this invention comprises the amino acid sequence of an Fc domain of an immunoglobulin. The Fc region of an immunoglobulin typically comprises the CH2 and CH3 domains of the heavy chain, as well as the hinge region. The Fc domain preferably comprises a sequence that is sufficient to allow the formation of a dimer (homodimer or heterodimer) between two fusion polypeptides of this invention. The Fc domain may be derived from an immunoglobulin of human or animal origin, such as, without limitation, rodent, equine or primate.

The Fc domain can be derived from an immunoglobulin of various serotypes, such as from an IgG, an IgA, an IgM, an IgD or an IgE. The sequence of the Fc domain preferably derives from an IgG. Examples of such Fc domain sequences are given in the present application. In particular, a specific example of the Fc domain of a human IgG is provided in SEQ ID NO: 2 (amino acid residues 235 to 452). A specific example of the Fc domain of a murine IgG is provided in SEQ ID NO: 6 (amino acid residues 120 to END). It should be understood that sequence variations may be tolerated within the Fc domain sequence, as long as the resulting sequence retains the ability to form dimers. The sequence of an Fc domain of an immunoglobulin can be obtained from the sequence of any known immunoglobulin according to techniques known in the art. It should be understood that the sequence of Fc domains may also be obtained from databasis and tested for their ability to dimerize in vitro.

A particular embodiment of this invention thus relates to a fusion polypeptide comprising a first polypeptide and a second polypeptide linked together through peptide linkage, wherein the first polypeptide is located N-ter of the second polypeptide, which is located C-ter of the fusion polypeptide, and wherein the first polypeptide comprises at least the sequence of an α1 domain of an HLA-G antigen and the second polypeptide comprises the sequence of an Fc domain of an immunoglobulin.

Specific examples of such fusion polypeptides of the invention are:
- HLA-Gα1-Fc of SEQ ID NO: 6 (or residues 21-351 thereof), and
- HLA-G6-Fc of SEQ ID NO: 2 (or residues 25-452 thereof).

In HLA-Gα1-Fc, the α1 domain of HLA-G is fused directly to the Fc domain of a mouse IgG2a. Intervening amino acid residues 111 to 119 are present between the two domains. Amino acid residues 1-20 correspond to the signal peptide sequence of the interleukin-2 protein. HLA-Gα1-Fc, in mature form, therefore comprises amino acid residues 21-351 of SEQ ID NO: 6.

In HLA-G6-Fc, the sequence of HLA-G6 is fused directly to the Fc domain of a human IgG2. Intervening amino acid residues 228-234 are present between the two domains. Amino acid residues 1-24 correspond to the signal peptide sequence of HLA-G. HLA-G6-Fc, in mature form, therefore comprises amino acid residues 25-452 of SEQ ID NO: 2.

As mentioned in the examples, both these polypeptides are able to promote graft tolerance in vivo. In particular, HLA-Gα1-Fc, which comprises only the α1 domain of HLA-G, was unexpectedly the most effective in delaying graft rejection in vivo.

As discussed above, the fusion polypeptides of this invention may comprise additional functional domain(s). In this respect, in a particular embodiment, the fusion polypeptides of this invention comprise a third polypeptide domain comprising the sequence of a β2 microglobulin. It is known that the HLA-G1 isoform of HLA-G forms a complex with β2 microglobulin at the cell surface. In order to mimic this complex, the invention proposes to include, in the fusion polypeptides, a sequence of the β2 microglobulin, arranged in a way allowing formation of a biologically active complex. When present, the polypeptide sequence of the β2 microblobulin is most preferably located on the N-terminal part of the fusion polypeptide, and it is linked to the first polypeptide sequence, according to the following scheme:

B2M sequence - HLA-G sequence - Fc domain

Furthermore, in a most preferred embodiment, the B2M sequence is linked to the HLA-G sequence through a spacer group, allowing proper refolding of the polypeptide. Most preferably, the spacer group comprises from 8 to 20 amino acid residues, more preferably from 8 to 15, even more preferably from 8 to 12. In a specific embodiment, the spacer group has the sequence (G4S)n, wherein n is 2 or 3.

A specific example of such a fusion polypeptide of this invention is :
- HLA-G1-B2M-Fc of SEQ ID NO: 4 (or residues 21-656 thereof).

In HLA-G1-B2M-Fc, the B2M sequence is fused to the αl-α2-α3 domains of HLA-G1 through a peptide linker (residues 120-134), and the HLA-G1 domain is linked to the Fc sequence of a human IgG2. Amino acid residues 1-20 correspond to the signal peptide sequence of B2M. HLA-G1-B2M-Fc, in mature form, therefore comprises amino acid residues 21-656 of SEQ ID NO: 4.

A further object of this invention is a dimer of a polypeptide as defined above. The dimer may be a homodimer, e.g., between two identical fusion polypeptides, or a heterodimer, e.g., between two distinct fusion polypeptides comprising an Fc domain.

The fusion polypeptides of this invention can be obtained using techniques known per se in the art, such as artificial synthesis, recombinant techniques, and/or combinations thereof. In a typical embodiment, as illustrated in the examples, the domains are produced by recombinant techniques, preferably directly in the form of a fusion polypeptide, starting from a chimeric coding polynucleotide.

In this respect, a further object of this invention is a nucleic acid molecule encoding a fusion polypeptide as defined above. The nucleic acid may be e.g., RNA or DNA, single- or double-stranded. It may be produced by techniques known per se in the art, such as genetic engineering, chemical or enzymatic synthesis, etc. In a particular embodiment, the nucleic acid further comprises a sequence encoding a leader peptide for secretion, operably linked to the sequence encoding the fusion polypeptide. As a result, expression of such a nucleic acid leads to the secretion of the fusion polypeptide by the selected host cell. The leader peptide may by of various origin, such as from human or mammalian genes, e.g., B2M, interleukin, HLA-, etc. Specific examples of nucleic acids of this invention are nucleic acid molecules comprising SEQ ID NO: 1, 3, or 5.

A further object of this invention also resides in a vector comprising a nucleic acid as defined above. The vector may be a cloning and/or expression vector, such as a plasmid, cosmid, phage, a viral vector, an artificial chromosome, etc. Specific examples of such vectors include pFUSE plasmids, pUC plasmids, pcDNA plasmids, pBR plasmids, retroviral vectors, adenoviral vectors, baculoviral vectors, lambda phage vectors, etc. The vector may comprise regulatory sequences, such as a promoter, a terminator, an origin of replication, etc. The vector may be used to produce polypeptides of this invention in vitro, by recombinant techniques, or directly in vivo, in gene therapy approaches.

A further object of this invention is a recombinant host cell comprising a nucleic acid or a vector as defined above. The host cell may be prokaryotic or eukaryotic. Examples of prokaryotic hosts include any bacteria, such as E. coli. Examples of eukaryotic cells include yeasts, fungi, mammalian cells, plant cells or insect cells. Recombinant cells of this invention may be prepared by transformation techniques known per se in the art, such as transfection, lipofection, electroporation, protoplast transformation, etc. These cells may be maintained and cultured in any suitable culture media.

Recombinant cells of this invention can be used e.g., to produce polypeptides of this invention in vitro or ex vivo, or as cell therapy products, to produce the polypeptides in vivo.

In this respect, an object of this invention also resides in a method of producing a polypeptide as disclosed above, the method comprising culturing a recombinant host cell of the invention under conditions allowing expression of the nucleic acid molecule, and recovering the polypeptide produced. The polypeptide may be recovered and/or purified using techniques known per se in the art, such as centrifugation, filtration, chromatographic techniques, etc.

Upon production, the polypeptides of this invention may be modified to improve their properties, for instance to improve their pharmaco-kinetic properties. In this respect, they may be modified to increase their stability or resistance to protease, such as by adding terminal protecting groups (e.g., amide, ester). They may also be coated on a carrier support to increase the polypeptide density.

A further object of this invention is a pharmaceutical composition comprising a polypeptide as defined above and, preferably, a pharmaceutically acceptable excipient or carrier.

A further object of this invention is a pharmaceutical composition comprising a nucleic acid as defined above and, preferably, a pharmaceutically acceptable excipient or carrier.

A further object of this invention is a pharmaceutical composition comprising a recombinant cell as defined above and, preferably, a pharmaceutically acceptable excipient or carrier.

Suitable excipients or carriers include any pharmaceutically acceptable vehicle such as buffering agents, stabilizing agents, diluents, salts, preservatives, emulsifying agents, sweeteners, etc. The excipient typically comprises an isotonic aqueous or non aqueous solution, which may be prepared according to known techniques. Suitable solutions include buffered solutes, such as phosphate buffered solution, chloride solutions, Ringer's solution, and the like. The pharmaceutical preparation is typically in the form of an injectable composition, preferably a liquid injectable composition, although other forms may be contemplated as well, such as tablets, gelules, capsules, syrups, etc. The compositions of this invention may be administered by a number of different routes, such as by systemic, parenteral, oral, rectal, nasal or vaginal route. They are preferably administered by injection, such as intravenous, intraarterial, intramuscular, intraperitoneal, or subcutaneous injection. Transdermal administration is also contemplated. The specific dosage can be adjusted by the skilled artisan, depending on the pathological condition, the subject, the duration of treatment, the presence of other active ingredients, etc. Typically, the compositions comprise unit doses of between 10ng and 100 mg of fusion polypeptide, more preferably between 1 µg and 50 mg, even more preferably between 100 µg and 50 mg.

The compositions of the present invention are preferably administered in effective amounts, i.e., in amounts which are, over time, sufficient to at least reduce or prevent disease progression. In this regard, the compositions of this invention are preferably used in amounts which allow the reduction of a deleterious or unwanted immune response in a subject.

As mentioned above, the fusion polypeptides of this invention have strong immune-regulatory activity and may be used to treat a variety of disease conditions associated with abnormal or unwanted immune response. More specifically, the polypeptides of this invention are suitable for treating immune-related disorders such as, particularly, organ or tissue rejection, inflammatory diseases or auto-immune diseases.

As disclosed in the experimental section, the polypeptides of this invention can substantially inhibit allogeneic or xenogenic graft rejection in vivo.

An object of the present invention thus resides in a polypeptide or composition as disclosed above for treating graft rejection.

A further object of this invention resides in a method of treating graft rejection in a subject, the method comprising administering to a subject in need thereof an effective amount of a composition as disclosed above.

The term treating designates for instance the promotion of the graft tolerance within the receiving subject. The treatment can be performed prior to, during and/or after the graft, and may be used as an alternative therapy to existing immunosuppressive agents or, as a combined therapy with actual immunosuppressive agents. The invention is applicable to allogenic, semi-allogenic or even xenogenic transplantation, and may be used for any type of transplanted organs or tissues including, without limitation, solid tissues, liquid tissues or cells, including heart, skin, kidney, liver, lung, liver-kidney, etc.

A further object of this invention is an improved method for transplanting an organ or tissue in a subject, the improvement comprising administering to the subject, prior to, during and/or after transplantation, an effective amount of a composition as disclosed above.

A further object of this invention is a method for promoting graft tolerance in a subject, the method comprising administering to the subject, prior to, during and/or after transplantation, an effective amount of a composition as disclosed above.

A further object of this invention is a method for reducing graft rejection in a subject, the method comprising administering to the subject, prior to, during and/or after transplantation, an effective amount of a composition as disclosed above.

A further object of the present invention resides in a polypeptide or composition as disclosed above for treating an auto-immune disease. The invention also resides in a method of treating an autoimmune disease in a subject, the method comprising administering to a subject in need thereof an effective amount of a composition as disclosed above. The autoimmune disease may be Rheumatoid arthritis, Crohn's disease or multiple sclerosis. In such disease conditions, the invention allows to reduce the deleterious immune response which is responsible for the pathology.

Another object of the present invention resides in a polypeptide or composition as disclosed above for treating an inflammatory disease.

A further object of this invention resides in a method of treating an inflammatory disease in a subject, the method comprising administering to a subject in need thereof an effective amount of a composition as disclosed above.

It should be understood that the amount of the composition actually administered shall be determined and adapted by a physician, in the light of the relevant circumstances including the condition or conditions to be treated, the exact composition administered, the age, weight, and response of the individual patient, the severity of the patient's symptoms, and the chosen route of administration. Therefore, the above dosage ranges are intended to provide general guidance and support for the teachings herein, but are not intended to limit the scope of the invention.

Further aspects and advantages of this invention will be disclosed in the following examples, which should be considered as illustrative and not limiting the scope of this application.

### EXAMPLES

### Materials and Methods

### Amplification by PCR

PCR were performed on a GeneAmp PCR System 9600 (Perkin Elmer) in a final volume of 50µl containing 20ng of DNA, 200nM of each primer, 200µM of dNTP (Invitrogen), 2.5µl of PCR Buffer 10X (Perkin Elmer), 2,5 Unit of Taq polymerase (Perkin Elmer) and 27µl of water.

Program used was the following :
DNA denaturation during 5 minutes at 94°C followed by 30 cycles of:
   30 seconds at 94°C
   30 seconds at 58°C
   1 minute at 72°C
At the end of the last cycle a 5 minutes step at 72°C was performed

### Vectors

pFUSE-hFcl and pFuse-rnFc2 vector were both purchased from the company InvivoGen.

### Enzymatic digestion

Enzymes restriction digestions were performed as recommended by the manufacturer (invitrogen). Typically, digestions were performed for 1 hour at 37°C with 1µg of DNA and 5 unit of restriction enzymes in the adequate buffer.

### Ligations

Ligation of PCR fragments into expression vector were performed with the T4 DNA ligase from Promega as recommended by the manufacturer. For HLA-G5-beta-2 microglobulin construction ligation of PCR fragment into pcDNA 3.1 D/V.5-His-Topo (Invitrogen) was performed directly with the 3.1 Directional TOPO^{®} Expression Kit (Invitrogen)

### Plasmid purification

Plasmid purification were performed with the GenElute™ Plasmid Midiprep (Sigma) as recommended by the manufacturer.

### Protein production

For production of the fusion protein, HEK293T or HELA cells were transfected by the diverse constructs with the lipofectamine method (invitrogen) and kept at 37°C, 5%CO2 in DMEM (Dulbco's Modified EagleMedium) supplemented with 10% foetal calf serum and 0.3M glutamine. After 48 hours supernatant were harvested, filtrated through 0.2 µM filter and then used for experiments or to prepare stocks.

### Example 1: Cloning and synthesis of HLA-G6-Fc

The HLA-G cDNA sequence of leader sequence, α1, α3 and intron 4 was amplified by PCR with template of pcDNA from HLA-G6. This sequence was amplified by PCR to introduce Age I and Xho I restriction sites with the following primers :
5'AAA**ACCGGT**ATGGTGGTCATGGCGCCCCG 3' (SEQ ID NO: 7)
   and
5'AAA**CTCGAG**AGGTCTTCAGAGAGGCTCCTGCTT' (SEQ ID NO: 8).

This amplified sequence was digested with Age I and Xho I restriction enzymes and then ligated into the pFUSE-hFc1 vector previously digested with Age I and Xho I. The resulting cDNA sequence is described in SEQ ID NO: 1 and the amino acid sequence is described in SEQ ID NO: 2.

The protein was produced as disclosed in the materials and methods.

### Example 2: Cloning and synthesis of HLA-G1-B2M-Fc

The sequence coding for the human Beta-2 microglobulin was amplified by PCR with primers B2M Sig **Mlu** I **Sph** IS cgtc **GCATGC ACGCGT** CG ATG TCT CGC TCC GTG GCC (SEQ ID NO: 9) and B2M-L-al AS **TCATGGAGTGGGAGCC** GGATCCGCCACCTCC GGATCCGCCACCTCC GGATCCGCCACCTCC CATGTCTCGATCCCACTT (SEQ ID NO: 10) that allowed to remove stop codon and to introduce a spacer corresponding to amino acid the sequence (GGGS)x2.
In parallel, the cDNA sequence corresponding to α1, α2 and α3 domain of HLA-G1 was amplified by PCR with primers HLA-Ga3 **Xho Sal** AS tatg **GTCGAC CTCGAG** CGC AGC TGC CTT CCA TCT CAG CAT GAG (SEQ ID NO: 11) and HLA-G a1 **Mlu Sph** S acgtc **GCATGC ACGCGT** CG GGC TTC CAC TCC ATG A (SEQ ID NO: 12) that allowed to remove peptide leader sequence and stop codon. Beta-2 microglobulin and α1, α2, α3 domain of HLA-G1 PCR fragments were both digested with Eag I restriction enzyme purified and ligated together. The Beta-2 microglobulin /α1, α2, α3 domain fusion sequence obtained was then digested with Mlu I and Xho I restriction enzyme and ligated in PGEMT/easy vector (Promega) previously digested with Mlu I and Xho I. This construction was then amplified by PCR with primers B2M Sig **Mlu I Sph I** IS cgtc **GCATGC ACGCGT** CG ATG TCT CGC TCC GTG GCC (SEQ ID NO: 13) and HLA-Ga3 **Xho Sal** AS tatg **GTCGAC CTCGAG** CGC AGC TGC CTT CCA TCT CAG CAT GAG (SEQ ID NO: 14). The amplified fragment obtained was then digested with Age I and Xho I and introduced into the cloning site Age I and Xho I of the vector pFUSE-hFcl in order to be in phase with cDNA coding for human Fc IgG2 (InVivogen, Toulouse, France). The resulting cDNA sequence is described in SEQ ID NO: 3 and the amino acid sequence is described in SEQ ID NO: 4.

The protein was produced as disclosed in the materials and methods.

### Example 3: Cloning and synthesis of HLA-Gα1-Fc

The cDNA sequence of HLA-G alpha-1 domain was amplified by PCR using primers 5'AAA **GAA TTC** GGG CTC CCA CTC CAT GAG GT 3' (SEQ ID NO: 15) and 5'AAA **GAT ATC** CCA CTG GCC TCG CTC TGG TTG3' (SEQ ID NO: 16). After restriction enzyme digestion of the alpha-1 PCR fragment and pFUSE-mFc2 vector (Invivogen) with restriction enzyme EcoRI and EcoRV, the alpha-1 PCR fragment was ligated into the pFUSE-mFc2 vector that contain the signal sequence of IL-2 and the Fc region of mouse IgG2a. The resulting cDNA sequence is described in SEQ ID NO: 5 and the amino acid sequence is described in SEQ ID NO: 6.

The protein was produced as disclosed in the materials and methods.

### Example 4: HLA-G/Fc fusion proteins form dimers

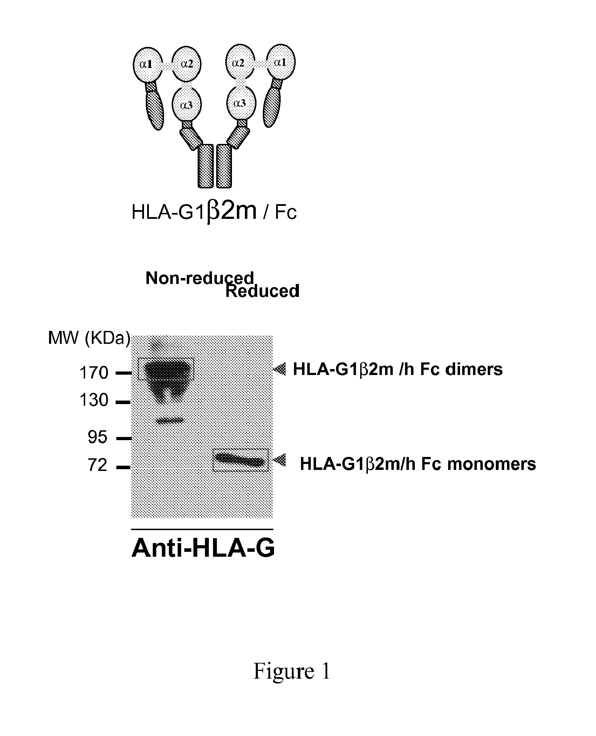

Figure 1 represents HLA-G-beta-2-microglobulin/Fc dimers ("non-reduced" (on the left)) and monomers ("reduced" (on the right)) protein migration by PolyAcrylamide Gel Electrophoresis. HLA-G/Fc proteins present in supernatant were immunoprecipitated with Protein G sepharose beads (GE Healthcare). Immunoprecipitates were washed three times with PBS 1X. Proteins were then eluted by incubation with sample buffer containing 10 mM of dithiothreitol ("reduced") or not ("non-reduced"), boiling, electrophoresed on polyacrylamide gels and transferred onto Hybond ECL nitrocellulose membranes (Amersham Pharmacia Biosciences). Following incubation with 5% non-fat milk in PBS 1X, the membrane was incubated overnight with anti-HLA-G (4H84) antibody and revealed using HorseRadish peroxydase-conjugated goat anti-mouse secondary antibody. Membranes were revealed with ECL detection system (Amersham Pharmacia Biosciences).

The results presented demonstrate the ability of HLA-G/Fc proteins of this invention to form dimers.

### Example 5: HLA-G-Fc fusion proteins induce signalling through ILT2 receptor

This example describe the effect of the HLA-G-Fc fusion proteins of this invention in a NFAT reporter cell assay, to determine binding to ILT2 receptor and subsequent signalling.

### Material

Sulfate latex beads 4%w/v 5/µm (Invitrogen)
AffmiPure Coat Anti-mouse IgG Fc Fragment 1.8mg/ml (Jackson ImmunoResearch)
AffmiPure Coat Anti-human IgG Fc Fragment 1.3mg/ml (Jackson ImmunoResearch)
Hela Negative Control
HLA- G1- b2m/hFc1 1.5 µg/ml
HLA- G6/Fc 0.5 µg/ml
NFATGFP reporter cells

### Method

NFATGFP reporter cells were cultured for 2 days before test. Briefly, reporter and HLA-G/Fc fusion protein coated beads were co-cultured at a 1:5 ratio for 16h and then analyzed for GFP expression by flow cytometry.

### Results

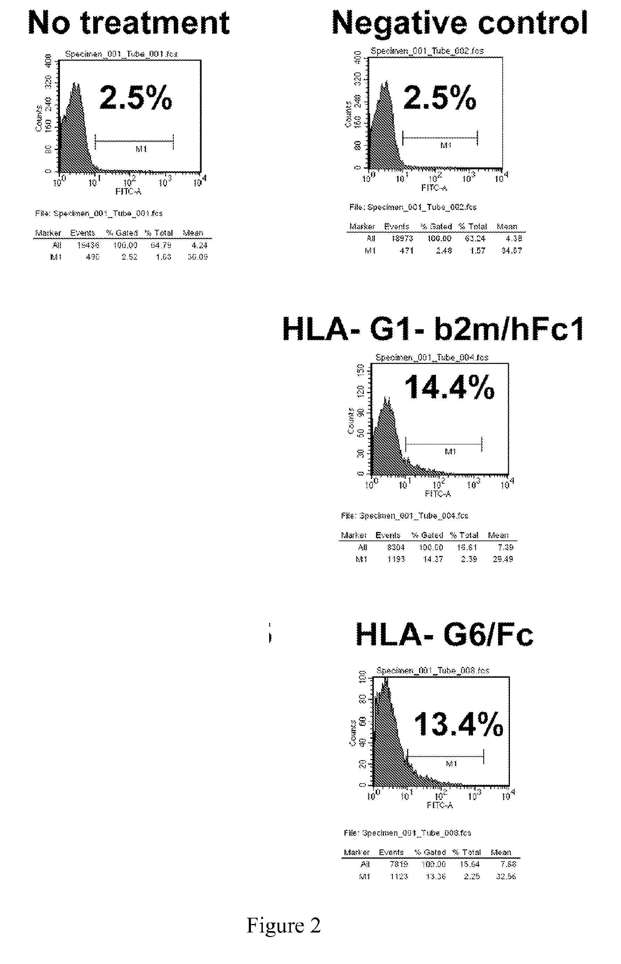

The results are depicted on Figure 2 and show that the fusion proteins are able to induce GFP expression, indicating they are functionally active.

### Example 6: Effect of HLA-G/Fc fusion proteins on allogeneic skin transplantation

### Materials

Sulfate latex beads 4%w/v 5um (Invitrogen)
AffiniPure Coat Anti-mouse IgG Fc Fragment 1.8mg/ml (Jackson ImmunoResearch)
AffmiPure Coat Anti-human IgG Fc Fragment 1.3mg/ml (Jackson ImmunoResearch)
HeLa Negative Control
HLA- G1- b2m/hFc1 1.5 µg/ml,
alphal/mfc2 1.5 µg/ml
HLA- G6/Fc 0.5 µg/ml.

### Method

For every HLA-G/Fc fusion protein, 10⁸ Sulfate latex beads were coated with 20µg/ml AffiniPure Coat Anti-mouse (or anti-human) IgG Fc Fragment 2hr at 37°C followed by 2hr incubation with BSA (2 mg/ml). After washing, the beads were incubated with 0.5µg/ml of HLA-G/Fc fusion proteins at 4°C for 16hr. Subsequently, the beads were washed 2 times by 1x PBS. 5ml of HLA-G/Fc fusion proteins (1µg/ml) was used for 5x 10⁶ sulfate latex beads. As a negative control, sulfate latex beads were prepared in an identical manner except that 1xPBS or HeLa Negative Control was used rather than HLA-G/Fc fusion proteins. Sulfate latex beads (5×10⁶) were injected intraperitoneally on the day before skin grafting.

Specific pathogen-free C57BL/6 (H-2b) mice and ILT4-transgcnic mice (H-2b) (8-10 weeks of age) were used as skin graft recipients throughout the study. Recipient mice received HLA-G-coupled microspheres, Donor skin was from MHC class II-disparate B6.CH-2bm12 (bm12, H-2b) mice. Allogeneic skin grafts have been performed by standard methods. Briefly, skin (1.0 cm²) from the tail of donor mice (12-14 weeks old) was grafted onto the flank of recipient, anesthetized mice. The graft was covered with gauze and plaster, which was removed on day 10. Grafts were scored daily until rejection (defined as 80% of grafted tissue becoming necrotic and reduced in size). All skin grafting survival data were tested by Kaplan Meier Survival Analysis.

### Results

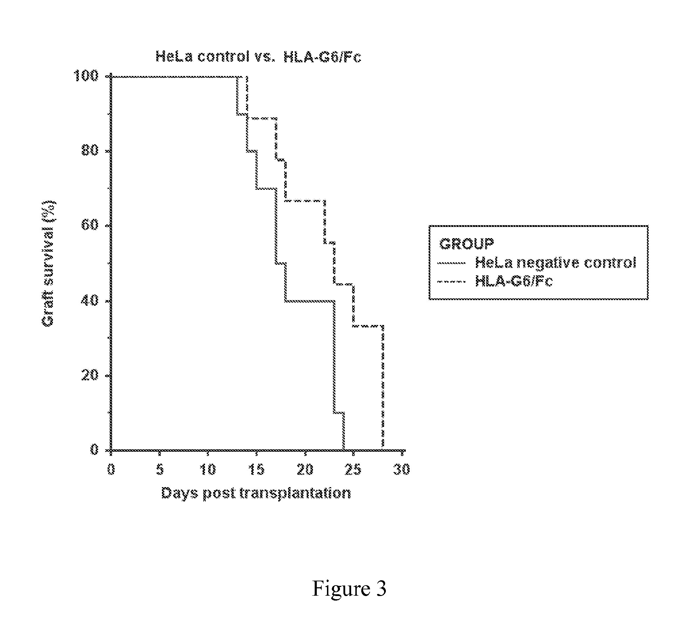

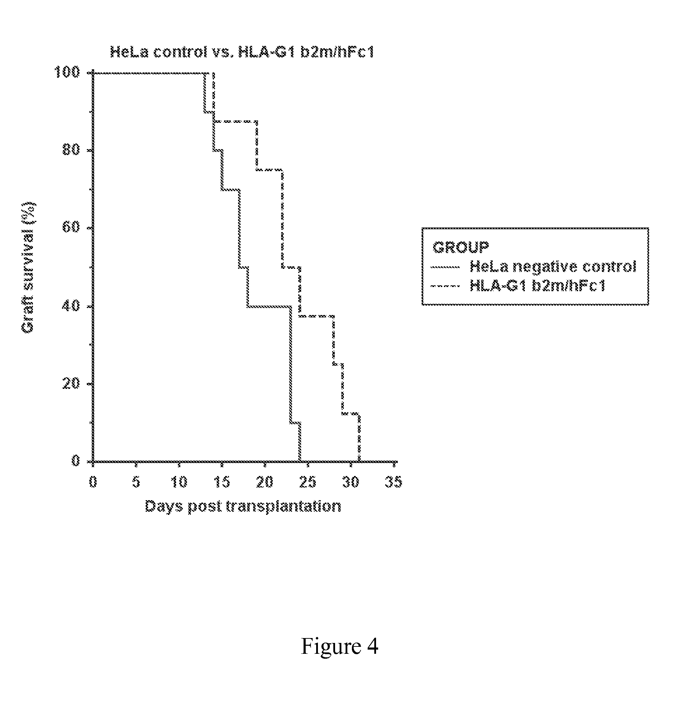

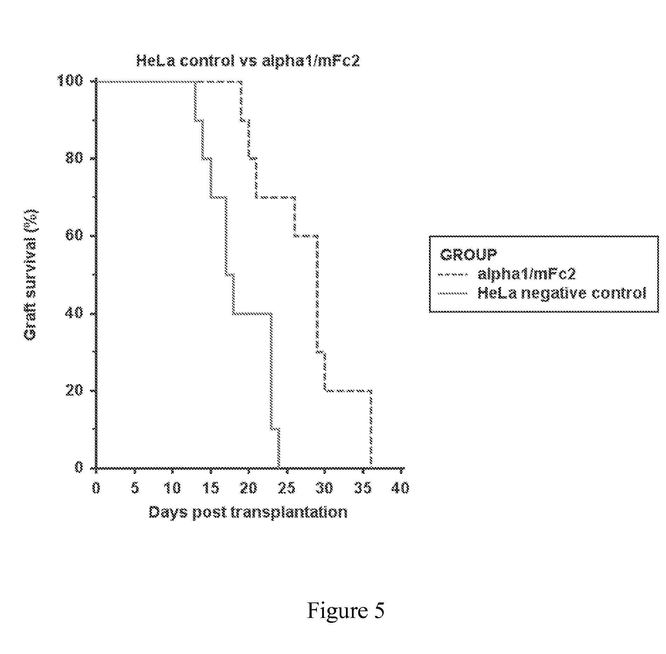

The results are depicted on Figures 3-5. They show that all fusion proteins were able to substantially improve graft tolerance in vivo. In particular, they show that the fusion proteins are able to improve by up to 50% the graft tolerance (see e.g., fig. 5), which is very surprising and substantial. It should be noted that each day of graft survival in the model corresponds to approximately at least one month of graft survival in human subjects, so that the proteins of this invention are believed to improve graft survival by at least 10 months in human subjects.

SEQUENCE LISTING

SEQ ID NO: 1 - cDNA Sequence for HLA-G6-hFc IgG2

DNA Sequence alpha-1 seq signal/alpha-1/alpha-3/intron-4/MCS/hFc IgG2

ACCGGTATGGTGGTCATGGCGCCCCGAACCCTCTTCCTGCTACTCTCGG
GGGCCCTGACCCTGACCGAGACCTGGGCGGGCTCCCACTCCATGAGGTA
TTTCAGCGCCGCCGTGTCCCGGCCCGGCCGCGGGGAGCCCCGCTTCATCGCC
ATGGGCTACGTGGACGACACGCAGTTCGTGCGGTTCGACAGCGACTCGGCG
TGTCCGAGGATGGAGCCGCGGGCGCCGTGGGTGGAGCAGGAGGGGCCAGA
GTATTGGGAAGAGGAGACACGGAACACCAAGGCCCACGCACAGACTGACA
GAATGAACCTGCAGACCCTGCGCGGCTACTACAACCAGAGCGAGGCCAACC
CCCCCAAGACACACGTGACCCACCACCCTGTCTTTGACTATGAGGCCACCCT
GAGGTGCTGGGCCCTGGGCTTCTACCCTGCGGAGATCATACTGACCTGGCA
GCGGGATGGGGAGGACCAGACCCAGGACGTGGAGCTCGTGGAGACCAGGC
CTGCAGGGGATGGAACCTTCCAGAAGTGGGCAGCTGTGGTGGTGCCTTCTG
GAGAGGAGCAGAGATACACGTGCCATGTGCAGCATGAGGGGCTGCCGGAG
CCCCTCATGCTGAGATGGAGTAAGGAGGGAGATGGAGGCATCATGTCTGTT
AGGGAAAGCAGGAGCCTCTCTGAAGACCTCTCGAGCACCATGGTTAGATCT
GTGGAGTGCCCACCTTGCCCAGCACCACCTGTGGCAGGACCTTCAGTC
TTCCTCTTCCCCCCAAAACCCAAGGACACCCTGATGATCTCCAGAACCC
CTGAGGTCACGTGCGTGGTGGTGGACGTGAGCCACGAAGACCCCGAGG
TCCAGTTCAACTGGTACGTGGACGGCATGGAGGTGCATAATGCCAAGA
CAAAGCCACGGGAGGAGCAGTTCAACAGCACGTTCCGTGTGGTCAGCG
TCCTCACCGTCGTGCACCAGGACTGGCTGAACGGCAAGGAGTACAAGT
GCAAGGTCTCCAACAAAGGCCTCCCAGCCCCCATCGAGAAAACCATCT
CCAAAACCAAAGGGCAGCCCCGAGAACCACAGGTGTACACCCTGCCCC
CATCCCGGGAGGAGATGACCAAGAACCAGGTCAGCCTGACCTGCCTGG
TCAAAGGCTTCTACCCCAGCGACATCGCCGTGGAGTGGGAGAGCAATG
GGCAGCCGGAGAACAACTACAAGACCACACCTCCCATGCTGGACTCCG
ACGGCTCCTTCTTCCTCTACAGCAAGCTCACCGTGGACAAGAGCAGGT
GGCAGCAGGGGAACGTCTTCTCATGCTCCGTGATGCATGAGGCTCTGC
ACAACCACTACACACAGAAGAGCCTCTCCCTGTCTCCGGGTAAATGA

SEQ ID NO: 2 : Protein sequence of HLA-G6-hFc IgG2

Signal sequence: amino acids 1-24
G6: amino acids 25-227
MCS: amino acids 228-234
Fc: amino acids 235-452

M V V M A P R T L F L L L S G A L T L T E T W A G S H S M R
Y F S A A V S R P G R G E P R F I A M G Y V D D T Q F V R F

D S D S A C P R M E P R A P W V E Q E G P E Y W E E E T R N
T K A H A Q T D R M N L Q T L R G Y Y N Q S E A N P P K T H
V T H H P V F D Y E A T L R C W A L G F Y P A E I I L T W Q
R D G E D Q T Q D V E L V E T R P A G D G T F Q K W A A V V
V P S G E E Q R Y T C H V Q H E G L P E P L M L R W S K E G
D G G I M S V R E S R S L S E D L S S T M V R S V E C P P C
P A P P V A G P S V F L F P P K P K D T L M I S R T P E V T
C V V V D V S H E D P E V Q F N W Y V D G M E V H N A K T K
P R E E Q F N S T F R V V S V L T V V H Q D W L N G K E Y K
C K V S N K G L P A P I E K T I S K T K G Q P R E P Q V Y T
L P P S R E E M T K N Q V S L T C L V K G F Y P S D I A V E
W E S N G Q P E N N Y K T T P P M L D S D G S F F L Y S K L
T V D K S R W Q Q G N V F S C S V M H E A L H N H Y T Q K S
L S L S P G K Stop

SEQ ID NO: 3: DNA sequence of HLA-G1 β2m hFc IgG2

seq signal B2m/Beta 2m/linker/α1/α2/α3/ hFc IgG2

ACCGGTATGTCTCGCTCCGTGGCCTTAGCTGTGCTCGCGCTACTCTCTCTTT
CTGGCCTGGAGGCTATCCAGCGTACTCCAAAGATTCAGGTTTACTCACGT
CATCCAGCAGAGAATGGAAAGTCAAATTTCCTGAATTGCTATGTGTCTG
GGTTTCATCCATCCGACATTGAAGTTGACTTACTGAAGAATGGAGAGAG
AATTGAAAAAGTGGAGCATTCAGACTTGTCTTTCAGCAAGGACTGGTCT
TTCTATCTCTTGTACTACACTGAATTCACCCCCACTGAAAAAGATGAGT
ATGCCTGCCGTGTGAACCATGTGACCTTGTCACAGCCCAAGATAGTTAA
GTGGGATCGAGACATGGGAGGTGGCGGATCCGGAGGTGGCGGATCCGGA
GGTGGCGGATCCGGCTCCCACTCCATGAGGTATTTCAGCGCCGCCGTGT
CCCGGCCCGGCCGCGGGGAGCCCCGCTTCATCGCCATGGGCTACGTGG
ACGACACGCAGTTCGTGCGGTTCGACAGCGACTCGGCGTGTCCGAGGA
TGGAGCCGCGGGCGCCGTGGGTGGAGGAGGAGGGGCCGGAGTATTGG
GAAGAGGAGACACGGAACACCAAGGCCCACGCACAGACTGACAGAATG
AACCTGCAGACCCTGCGCGGCTACTACAACCAGAGCGAGGCCAGTTCT
CACACCCTCCAGTGGATGATTGGCTGCGACCTGGGGTCCGACGGACGC
CTCCTCCGCGGGTATGAACAGTATGCCTACGATGGCAAGGATTACCTC
GCCCTGAACGAGGACCTGCGCTCCTGGACCGCAGCGGACACTGCGGCT
CAGATCTCCAAGCGCAAGTGTGAGGCGGCCAATGTGGCTGAACAAAGG
AGAGCCTACCTGGAGGGCACGTGCGTGGAGTGGCTCCACAGATACCTG
GAGAACGGGAAGGAGATGCTGCAGCGCGCGGACCCCCCCAAGACACA
CGTGACCCACCACCCTGTCTTTGACTATGAGGCCACCCTGAGGTGCTG
GGCCCTGGGCTTCTACCCTGCGGAGATCATACTGACCTGGCAGCGGGA
TGGGGAGGACCAGACCCAGGACGTGGAGCTCGTGGAGACCAGGCCTG
CAGGGGATGGAACCTTCCAGAAGTGGGCAGCTGTGGTGGTGCCTTCTG
GAGAGGAGCAGAGATACACGTGCCATGTGCAGCATGAGGGGCTGCCG
GAGCCCCTCATGCTGAGATGGAAGGCAGTTGCCTCGAGCACCATGGTTAG
ATCTGTGGAGTGCCCACCTTGCCCATCGAGCACCATGGTTAGATCTGTGGAGTG
CCCACCTTGCCCAGCACCACCTGTGGCAGGACCTTCAGTCTTCCTCTTC
CCCCCAAAACCCAAGGACACCCTGATGATCTCCAGAACCCCTGAGGTC

ACGTGCGTGGTGGTGGACGTGAGCCACGAAGACCCCGAGGTCCAGTTC
AACTGGTACGTGGACGGCATGGAGGTGCATAATGCCAAGACAAAGCCA
CGGGAGGAGCAGTTCAACAGCACGTTCCGTGTGGTCAGCGTCCTCACC
GTCGTGCACCAGGACTGGCTGAACGGCAAGGAGTACAAGTGCAAGGTC
TCCAACAAAGGCCTCCCAGCCCCCATCGAGAAAACCATCTCCAAAACCA
AAGGGCAGCCCCGAGAACCACAGGTGTACACCCTGCCCCCATCCCGGG
AGGAGATGACCAAGAACCAGGTCAGCCTGACCTGCCTGGTCAAAGGCT
TCTACCCCAGCGACATCGCCGTGGAGTGGGAGAGCAATGGGCAGCCGG
AGAACAACTACAAGACCACACCTCCCATGCTGGACTCCGACGGCTCCTT
CTTCCTCTACAGCAAGCTCACCGTGGACAAGAGCAGGTGGCAGCAGGG
GAACGTCTTCTCATGCTCCGTGATGCATGAGGCTCTGCACAACCACTAC
ACACAGAAGAGCCTCTCCCTGTCTCCGGGTAAATGA

SEQ ID NO: 4 : Protein HLA-G1 β2m hFc IgG2

seq signal B2m: amino acids 1-20
Beta 2m: amino acids 21-119
Linker: amino acids 120-134
α1/α2/α3: amino acids 135-412
hFc IgG2 amino acids 413-END

























SEQ ID NO: 5 : DNA Sequence of seq signal IL2/alpha-1/MCS/Mouse IgG2a Fc

ATGTACAGGATGCAACTCCTGTCTTGCATTGCACTAAGTCTTGCACTTGTCA
CGAATTCGGGCTCCCACTCCATGAGGTATTTCAGCGCCGCCGTGTCCCGGCC
CGGCCGCGGGGAGCCCCGCTTCATCGCCATGGGCTACGTGGACGACACGCA
GTTCGTGCGGTTCGACAGCGACTCGGCGTGTCCGAGGATGGAGCCGCGGGC
GCCGTGGGTGGAGCAGGAGGGGCCAGAGTATTGGGAAGAGGAGACACGGA
ACACCAAGGCCCACGCACAGACTGACAGAATGAACCTGCAGACCCTGCGCG
GCTACTACAACCAGAGCGAGGCCAGTGGGATATCGGCCATGGTTAGATCTCCC
AGAGGGCCCACAATCAAGCCCTGTCCTCCATGCAAATGCCCAGCACCT
AACCTCTTGGGTGGACCATCCGTCTTCATCTTCCCTCCAAAGATCAAGG
ATGTACTCATGATCTCCCTGAGCCCCATAGTCACATGTGTGGTGGTGGA
TGTGAGCGAGGATGACCCAGATGTCCAGATCAGCTGGTTTGTGAACAA
CGTGGAAGTACACACAGCTCAGACACAAACCCATAGAGAGGATTACAA
CAGTACTCTCCGGGTGGTCAGTGCCCTCCCCATCCAGCACCAGGACTG
GATGAGTGGCAAGGAGTTCAAATGCAAGGTCAACAACAAAGACCTCCC
AGCGCCCATCGAGAGAACCATCTCAAAACCCAAAGGGTCAGTAAGAGC
TCCACAGGTATATGTCTTGCCTCCACCAGAAGAAGAGATGACTAAGAAA
CAGGTCACTCTGACCTGCATGGTCACAGACTTCATGCCTGAAGACATTT
ACGTGGAGTGGACCAACAACGGGAAAACAGAGCTAAACTACAAGAACA
CTGAACCAGTCCTGGACTCTGATGGTTCTTACTTCATGTACAGCAAGCT
GAGAGTGGAAAAGAAGAACTGGGTGGAAAGAAATAGCTACTCCTGTTC
AGTGGTCCACGAGGGTCTGCACAATCACCACACGACTAAGAGCTTCTC
CCGGACTCCGGGTAAATGA

SEQ ID NO: 6:
seq signal IL2: amino acids 1-20
alpha-1: amino acids 21-110
MCS: amino acids 111-119
Mouse IgG2a Fc: amino acids 120-END

M Y R M Q L L S C I A L S L A L V T N S G S H S M R Y F S A
A V S R P G R G E P R F I A M G Y V D D T Q F V R F D S D S
A C P R M E P R A P W V E Q E G P E Y W E E E T R N T K A H
A Q T D R M N L Q T L R G Y Y N Q S E A S G I S A M V R S P
R G P T I K P C P P C K C P A P N L L G G P S V F I F P P K
I K D V L M I S L S P I V T C V V V D V S E D D P D V Q I S
W F V N N V E V H T A Q T Q T H R E D Y N S T L R V V S A L
P I Q H Q D W M S G K E F K C K V N N K D L P A P I E R T I

S K P K G S V R A P Q V Y V L P P P E E E M T K K Q V T L T
C M V T D F M P E D I Y V E W T N N G K T E L N Y K N T E P
V L D S D G S Y F M Y S K L R V E K K N W V E R N S Y S C S
V V H E G L H N H H T T K S F S R T P G K Stop

## Claims

1. A fusion polypeptide comprising a first polypeptide comprising the sequence of a domain of an HLA-G antigen linked to a second polypeptide comprising the sequence of an Fc domain of an immunoglobulin.

2. The fusion polypeptide of claim 1, wherein the first polypeptide is N-ter of the fusion polypeptide, and the second polypeptide is C-ter.

3. The fusion polypeptide of claim 1 or 2, wherein the second polypeptide comprises the sequence of a human or murine immunoglobulin.

4. The fusion polypeptide of any one of the preceding claims, wherein the immunoglobulin is an IgG, an IgA, an IgM or an IgE, preferably an IgG.

5. The fusion polypeptide of any one of the preceding claims, wherein the HLA-G antigen is a human HLA-G antigen.

6. The fusion polypeptide of any one of the preceding claims, wherein the domain of said HLA-G antigen comprises at least the α1 domain.

7. The fusion polypeptide of any one of the preceding claims, wherein said first polypeptide is selected from :
- the amino acid sequence of the α1, α2 and α3 domains of an HLA-G antigen;
- the amino acid sequence of the α1 and α3 domains of an HLA-G antigen; or
- the amino acid sequence of the α1 and α2 domains of an HLA-G antigen.

8. The fusion polypeptide of anyone of the preceding claims, wherein said first and second polypeptides are linked directly or through a spacer.

9. The fusion polypeptide of anyone of the preceding claims, which further comprises a third polypeptide comprising the sequence of a β2 microglobulin.

10. A fusion polypeptide, which is selected from:
- HLA-Gl-B2M-Fc comprising SEQ ID NO: 4, or amino acid residues 21-656 thereof,
- HLA-Gal-Fc comprising SEQ ID NO: 6, or amino acid residues 21-351 thereof; and
- HLA-G6-Fc comprising SEQ ID NO: 2, or amino acid residues 25-452 thereof.

11. A nucleic acid molecule encoding a fusion polypeptide of any one of claims 1 to 10.

12. A vector comprising a nucleic acid molecule of claim 11.

13. A recombinant host cell comprising a nucleic acid molecule of claim 11 or a vector of claim 12.

14. A method of producing a polypeptide of any one of claims 1 to 10, comprising culturing a recombinant host cell of claim 13 under conditions allowing expression of the nucleic acid molecule, and recovering the polypeptide produced.

15. A dimer of a polypeptide of any one of claims 1 to 10.

16. A pharmaceutical composition comprising a polypeptide of any one of claims 1 to 10.

17. The pharmaceutical composition of claim 16, for treating organ or tissue rejection.

18. The pharmaceutical composition of claim 16, for treating an inflammatory disease or an auto-immune disease.
